# EUROPÄISCHE PATENTSCHRIFT

(11) **EP 1 279 364 B1**
(45) Veröffentlichungstag und Bekanntmachung des Hinweises auf die Patenterteilung: **12.05.2004**
(21) Anmeldenummer: 02015977.8
(22) Anmeldetag: 18.07.2002
(51) Int. Cl.: A61B 1/07, A61B 5/00

(54) **Vorrichtung zur bildgebenden und spektroskopischen Diagnose von Gewebe**
Apparatus for imaging and spectroscopic diagnosis of tissue
Appareil d'imagerie et de diagnostique spectroscopique des tissus

(30) Priorität: 25.07.2001 DE 10136191
(43) Veröffentlichungstag der Anmeldung: 29.01.2003
(73) Patentinhaber: Richard Wolf GmbH, 75438 Knittlingen (DE)
(72) Erfinder: Weber, Bernd Claus, 76133 Karlsruhe (DE); Goll, Thomas, 75438 Knittlingen (DE); Schmidt, Olaf, 71665 Vaihingen/Enz (DE); Eidner, Phillip, 75015 Bretten-Büchig (DE); Müller, Stefan, 75015 Bretten-Diedelsheim (DE); Delgado Pereira, Nicolas, 75433 Maulbronn (DE); Freitag, Lutz, Dr., 58675 Hemer (DE)
(74) Vertreter: Wilcken, Thomas, Dipl.-Ing.

(56) Entgegenhaltungen:
- DE-A- 10 031 818
- DE-A- 10 055 725
- DE-A- 19 612 536
- US-A- 5 042 494
- US-A- 5 507 287
- US-A- 6 161 035

## Beschreibung

Die Erfindung betrifft eine Vorrichtung zur bildgebenden und spektroskopischen Diagnose von Gewebe unter alternativer oder kombinierter Anwendung von drei Diagnosemethoden, nämlich einem Modus A zur bildgebenden Weißlichtdiagnose, einem Modus B zur bildgebenden Fluoreszenzdiagnose und einem Modus C zur fluoreszenzspektroskopischen Diagnose, wobei die Vorrichtung ein Leuchtmittel aufweist, dessen Licht als Strahlenbündel über einen Strahlengang in einen zu einem Endoskop führenden Lichtleiter eingekoppelt wird.

Diagnosevorrichtungen der gattungsgemäßen Art werden zur großflächigen bildgebenden Weißlichtuntersuchung (Modus A), zur großflächigen bildgebenden Fluoreszenzdiagnose (Modus B) und zur punktuellen Fluoreszenzspektroskopie (Modus C), d.h. zur spektroskopischen Untersuchung der Fluoreszenz von kleinsten Gewebebereichen (optische Biopsie), benötigt. Die spektroskopische Untersuchung bezieht sich dabei auf die Gewebebereiche, die vorab bei der bildgebenden Fluoreszenz- oder bei der bildgebenden Weißlichtdiagnose auffällig geworden sind.

Für den untersuchenden Arzt ist es wünschenswert, zunächst eine gewöhnliche großflächige Weißlichtdiagnose (erster Diagnosemodus A im Sinne der vorliegenden Erfindung) durchzuführen, um sich zunächst unter Weißlicht einen Überblick über das zu untersuchende Gewebe zu verschaffen und eine Vorabdiagnose zu erstellen. Hierbei wird, soweit dieses unter Weißlicht sichtbar ist, nach entzündetem, malignem und frühmalignem Gewebe gesucht. Die Untersuchung erfolgt dabei durch großflächiges Betrachten des Gewebes.

Hierfür muss sichergestellt sein, dass der Objektfeldwinkel und dementsprechend auch der Beleuchtungswinkel groß genug sind.

Bei der konventionellen Weißlichtdiagnose ist jedoch häufig frühmalignes Gewebe nicht von benignem Gewebe zu unterscheiden, weshalb es nicht aufgefunden werden kann. Die Weißlichtdiagnose hat hierfür eine unzureichende Sensitivität. Dementsprechend kann eine lebensrettende oder zumindest deutlich lebensverlängernde Therapie nicht durchgeführt werden.

Hier stellt die bekannte bildgebende Fluoreszenzdiagnose (Modus B im Sinne der vorliegenden Erfindung) einen entscheidenden Vorteil dar. Bei entsprechendem Objektfeld- und Beleuchtungswinkel, die identisch mit denen bei der Weißlichtdiagnose, also ausreichend groß, sein können, kann der Arzt im Modus der bildgebenden Fluoreszenzdiagnose das Gewebe großflächig einsehen, und er wird durch Intensitäts- und Farbunterschiede zwischen benignem und (früh-) malignem Gewebe schnell auf verdächtige Stellen aufmerksam.

In der Literatur ist nachgewiesen worden, dass die Sensitivität bei der Krebsfrüherkennung durch die zusätzliche bildgebende Fluoreszenzdiagnose gegenüber der alleinigen Weißlichtdiagnose erheblich gesteigert werden kann. Allerdings sind bei der Spezifität noch Defizite zu beobachten. So zeichnen sich beispielsweise bei der Autofluoreszenzdiagnose manche (früh-) maligne Läsionen lediglich durch einen Rückgang der Fluoreszenzintensität aus, die Farbverschiebung zum roten Spektralbereich hin kann aber aufgrund der unter Umständen gegenüber dem gesunden Gewebe stark reduzierten Fluoreszenz kaum noch oder gar nicht mehr wahrgenommen werden. Die verdächtige Stelle scheint lediglich dunkel, und Farben sind dadurch kaum mehr zu erkennen. Eine Helligkeitsanhebung führt lediglich dazu, dass das umgebende gesunde Gewebe überstrahlt wirkt und deshalb die Farbveränderungen, gerade bei kleinen verdächtigen Stellen, dann auch nicht wahrgenommen werden können.

Diese Läsionen können dementsprechend kaum bzw. nicht von gesundem Gewebe unterschieden werden, welches beispielsweise allein aufgrund einer veränderten Oberflächenstruktur (z.B. morphologisch stark strukturiertes Gewebe, weiches als "Lichtfalle" wirkt und damit den gesunden Gewebebereich gleichfalls dunkel wirken läßt) ebenfalls weniger Fluoreszenzlicht als morphologisch normal strukturiertes gesundes Gewebe dem Beobachter zuführt.

Unsicherheiten bei der Beurteilung solchen Gewebes machen in all solchen Fällen eine Probeentnahme obligatorisch, um möglichst alle potentiellen frühmalignen bzw. malignen Herde zu erfassen. Dadurch steigt aber die Falsch-Positiv-Rate, d.h. die Spezifität sinkt. In gleichem Maße steigen Zeitaufwand und die Kosten der Untersuchung durch den höheren Zeitaufwand und zusätzlich erforderliche Biopsien.

Eine Aufnahme und Darstellung des Fluoreszenzspektrums (Modus C im Sinne der vorliegenden Erfindung) gibt hier detailliertere Auskunft über den Zustand des betreffenden Gewebes durch Aufzeichnung und Aus- und Bewertung der spektralen Zusammensetzung der Fluoreszenzemission des zweifelhaften Gewebes, während die vorher durchgeführte bildgebende Fluoreszenzdiagnose neben den Informationen zur wechselnden Fluoreszenzintensität lediglich einen integralen Farbeindruck des betreffenden Gewebes als Ergebnis einer vom Auge durchgeführten Summierung aller Spektralanteile (unter Berücksichtigung der spektralen Augenempfindlichkeitskurve) wiedergeben konnte. Einzelheiten hierzu sind in der DE 196 12 536 A1 beschrieben.

Die sogenannte optische Biopsie in Form einer punktuellen fluoreszenzspektroskopischen Untersuchung erlaubt damit ohne eine Probeentnahme mit anschließender zytologischer Untersuchung eine genauere und bessere Beurteilung und Vorselektion des Gewebes als dies allein mit der großflächigen bildgebenden Fluoreszenzdiagnose möglich ist.

Durch diese Vorselektion müssen deshalb weniger Gewebestellen biopsiert und dem Pathologen zugeführt werden. Die Auswertung des Fluoreszenzspektrums und die Beurteilung des betrachteten punktuell kleinen Gewebebereichs, d.h. die Auswertung und Beurteilung der Spektralkurve, kann entweder durch den Arzt selbst oder durch einen an das Spektrometer angeschlossenen Computer vorgenommen werden, der bestimmte Punkte des emittierten Fluoreszenzspektrums mit den entsprechenden Punkten von zuvor aufgenommenem und abgespeichertem gesunden Gewebe des untersuchten Patienten vergleicht. Mit dieser optischen Biopsie können zum Teil auch Randbereiche von frühmalignem bzw. malignem Gewebe besser bzw. mit größerer Sicherheit vom umgebenden gesunden Gewebe abgegrenzt werden.

Bezüglich der Fluoreszenzspektroskopie ist aber Folgendes zu beachten. Das für die Auswertung und Bewertung erzeugte und ggf. auf einem Monitor dargestellte Spektrum ist das gemittelte Ergebnis des gesamten vom Spektrometer detektierten (Flächen-) Bereichs. Die Größe des Gewebebereichs, welcher dem Spektrometer Fluoreszenzsignale zuführt, wird im Wesentlichen durch zwei Komponenten bestimmt. Zum einen durch den Fluoreszenzanregungsstrahlenkegel, der wiederum nach oben durch die numerische Apertur der Anregungsfaser (Faserbündel) begrenzt ist, sofern auf zusätzliche optische Komponenten verzichtet wird, sowie auch durch die Größe des Einkoppelstrahlungskegels der Lichtquelle in die Faser (Faserbündel), zum anderen aber auch durch die numerische Apertur der detektierenden Faser (Faserbündel) bzw. durch sich evt. daran anschließende aperturbegrenzende Vorrichtungen.

Soll nun das örtliche Auflösungsvermögen bei der Suche nach Frühkarzinomen möglichst groß sein, d.h. sollen also auch möglichst kleine Läsionen erkannt werden und spektroskopisch nicht im umgebenden gesunden Gewebe untergehen, muss auch der bei der Fluoreszenzspektroskopie angeregte Bereich und/oder der detektierte Bereich möglichst klein sein. Andernfalls, wenn der angeregte Gewebebereich und der detektierte Gewebebereich wesentlich größer sind als die verdächtige Stelle, kann es passieren, dass der Verlauf der auszuwertenden Spektralkurve im Wesentlichen durch das das kranke Gewebe umgebende gesunde Gewebe bestimmt wird, weil dies für die Erzeugung der Spektralkurve anteilsmäßig einen entsprechenden größeren Gewebeflächen- und damit Fluoreszenzbeitrag liefert. Die Fluoreszenzinformation der kranken Gewebestelle geht dann in der Fluoreszenzinformation des benachbarten gesunden Gewebes unter, und die kranke Stelle wird nicht als solche erkannt.

Diese Aspekte sind vor allen Dingen bei der spektroskopischen Autofluoreszenzdiagnose (Modus C) zu berücksichtigen, bei der die Fluoreszenz des gesunden Gewebes die Fluoreszenz des malignen Gewebes hinsichtlich der Intensität deutlich dominiert. Nur bei der medikamenteninduzierten Fluoreszenz spielen diese Überlegungen eine geringere Rolle, da sich dort im Idealfall nur das frühmaligne bzw. maligne Gewebe mit dem fluoreszierenden Medikament anreichert und so das das kranke Gewebe umgebende gesunde Gewebe relativ schwach oder gar nicht fluoresziert. Kritischer ist die Situation auch bei der medikamenteninduzierten Fluoreszenz dann, wenn beispielsweise für eine Kontraststeigerung remittiertes Anregungslicht detektiert wird.

Insgesamt bedeutet dies, dass sowohl für die konventionelle Weißlichtdiagnose als auch für die bildgebende Fluoreszenzdiagnose distal große Beleuchtungs- bzw. Anregungsstrahlenkegel und gleichermaßen große Objektfeldwinkel des Bildkanals erstrebenswert sind. Bei der punktuellen Fluoreszenzspektroskopie hingegen (Modus C) muss der Anregungslichtkegel am distalen Ende des Endoskops oder die numerische Apertur der Detektionsfaser oder eine evt. zusätzlich zu dem Spektrometer eingebrachte aperturmanipulierbare Vorrichtung entsprechend dem gewünschten Ortsauflösungsvermögen bei der Krebsfrüherkennung ausreichend klein eingestellt werden können.

Soll aber dieser Gewebebereich, von welchem dem Spektrometer Fluoreszenzsignale zugeführt werden, dem untersuchenden Arzt auch sichtbar gemacht werden, was im Falle der Autofluoreszenz im Gegensatz zur medikamenteninduzierten Fluoreszenz aus oben genannten Gründen von eminenter Wichtigkeit ist, so kann dies nur durch einen diesem Gewebebereich entsprechenden kleinen Anregungslichtfleck erfolgen, der sich dann z.B. farblich vom umgebenden, konventionell weiß beleuchteten Gewebe abhebt. Beispielsweise kann sich der Anregungslichtfleck als "blauer Anregungspunkt" auf dem verdächtigen Gewebe vom umgebenden weiß beleuchteten Gewebe unterscheiden. Der Arzt kann also dadurch und nur so feststellen, von welchem Bereich bzw. Bereichsanteil des großflächig eingesehenen Gewebeareals, das weiß beleuchtet ist, die Spektrometerkurve gebildet wird.

Ein System, das die Anwendung aller drei genannten Diagnosemethoden ermöglicht, ist aus der DE 196 18 963 C2 bekannt. Die konventionelle Weißlichtuntersuchung (Modus A) und die bildgebende Fluoreszenzdiagnose (Modus B) erfolgen mit einem ersten apparativen Aufbau. Um noch zusätzlich punktuelle Fluoreszenzspektroskopie (Modus C) betreiben zu können, wird in der DE 196 18 963 C2 eine Zusatzvorrichtung vorgeschlagen, die als Aufsatz für das Endoskop ausgebildet ist. Die Zusatzvorrichtung enthält einen Strahlteiler, der dafür sorgt, dass nicht mehr das gesamte vom Endoskopbildleiter zur Verfügung gestellte Licht dem beobachtenden Auge bzw. der angeschlossenen Kamera zugeführt wird, sondern nun ein Teil dieses Lichts ausgekoppelt wird, um einem Spektrometer zugeführt zu werden.

Dieses Vorgehen bringt folgende Nachteile mit sich. Die auf das Endoskop aufgesteckte Zusatzvorrichtung erhöht sowohl das Gewicht als auch die geometrischen Abmessungen des aufgerüsteten Diagnoseinstruments und beeinträchtigt dadurch dessen Handhabbarkeit. Auf zusätzlich am Endoskop fixierte Elemente muss daher zwecks bequemer Handhabung möglichst verzichtet werden.

Problematisch ist beim bekannten Stand der Technik weiterhin folgender Umstand. Generell gilt für die Autofluoreszenzdiagnose, dass die geringe Intensität des Autofluoreszenzlichts fast immer ein Problem darstellt, was, sofern auf unhandliche und insofern nachteilige Bildverstärker verzichtet wird, meist in der Notwendigkeit der Integration mehrerer Videohalbbilder resultiert. Das wiederum führt zu Qualitätseinbußen bei der Bildwiedergabe, die sich in einer reduzierten Bildwiederholrate äußern. Wird schließlich, wie im Falle des vorbekannten Geräts, auch noch Autofluoreszenzlicht abgekoppelt, um diesen Anteil dem Spektrometer zuzuführen, wird die Problematik der geringen Menge an Autofluoreszenzlicht im Direkteinblick bzw. Kamerakanal weiter verschärft, d.h., die ohnehin schon beeinträchtigte Bildqualität beim bildgebenden Fluoreszenzdiagnoseverfahren wird durch die in nun noch stärkerem Umfang erforderliche Bildintegration weiter verschlechtert.

Bei der Zusatzvorrichtung gemäß der DE 196 18 963 C2 wird der Durchmesser des sogenannten zentralen Spektraldetektionsfeldes, also der Durchmesser jenes Gewebebereichs, dessen Fluoreszenz dem Spektrometer zugeführt wird und welche damit die Basis für das erzeugte Spektrum bildet, durch die Brennweite der Linse in der Zusatzvorrichtung einerseits und durch den Durchmesser der angeschlossenen Faser bzw. des Faserbündels als Verbindung zwischen dem Endoskop und dem Spektrometer andererseits bestimmt. Dementsprechend ist eine Änderung dieses Durchmessers des Spektraldetektionsfeldes nicht ohne Aufwand realisierbar und während einer Untersuchung am Patienten praktisch nicht machbar, zumindest dann nicht, wenn die Möglichkeit bestehen soll, häufige Verstellungen vornehmen zu können.

Die Fluoreszenzspektroskopie erfolgt bei der Lösung gemäß der DE 196 18 963 C2 simultan zur bildgebenden Fluoreszenzdiagnose, da ja gerade während des bildgebenden Fluoreszenzdiagnoseverfahrens ein Teil des Fluoreszenzlichts abgekoppelt wird. Für die Lokalisierung bzw. Ortsauffindung der Fluoreszenzspektroskopiestelle bzw. der Region, in der sich diese Spektroskopiestelle befindet, dient das Bild, das durch die bildgebende Fluoreszenzdiagnose geliefert wird. Das Spektraldetektionsfeld befindet sich zentral, also in der Mitte des durch die bildgebende Fluoreszenzdiagnose gelieferten Bildes. Bei der Autofluoreszenzdiagnose sind jedoch, wie bereits dargestellt, die Fluoreszenzsignale relativ schwach, und aufgrund der daraus resultierenden Notwendigkeit der Integration mehrerer Videohalbbilder beim Kameraeinsatz ist die Bildqualität auf dem Monitor in keiner Weise vergleichbar mit derjenigen bei der Weißlichtdiagnose. Aus diesem Grund ist es vorteilhaft, sich bei der Durchführung der Fluoreszenzspektroskopie am Weißlichtbild statt am Bild, das durch die bildgebende Fluoreszenzdiagnose geliefert wird, zu orientieren. Der Gewebebereich, der die potentielle Läsion, bei der die punktuelle Fluoreszenzspektroskopie durchgeführt wird, umgibt, sollte für eine bestmögliche Orientierung des untersuchenden Arztes in gewohnt guter Weißlichtbildqualität dargestellt werden. Dies ist bei der aus der DE 196 18 963 C2 bekannten Vorrichtung nicht der Fall.

Nachteilig bei der vorbekannten Vorrichtung ist weiterhin die Tatsache, dass der untersuchende Arzt tatsächlich nicht über den exakten Ort und vor allem nicht über die exakte Größe der Gewebestelle (Durchmesser), die die Information für die punktuelle Fluoreszenzspektroskopie liefert, informiert ist. Der spektroskopisch untersuchte Ort liegt zwar aufgrund der Anordnung der Optik im Zentrum des am Okulareinblick einzusehenden Bildes, was der Arzt weiß, aber eben nicht explizit sieht. Dieses Zentrum ist jedoch aufgrund optischer Täuschungen, die aus dem Bildinhalt resultieren können, nicht immer leicht zu bestimmen. Ähnliches gilt für die Größe und den Durchmesser der spektroskopisch ausgewerteten Gewebestelle. Konfus wird die Situation diesbezüglich insbesondere dann, wenn noch oben beschriebene Maßnahmen zur Durchmesserveränderung vorgenommen werden.

Ein anderes System ist aus dem Buch "Bronchoscopy", U. B. S. Prakash, Mayo Foundation, Raven Press Ltd., New York, Chapter 15, bekannt. Auch hier ist wie in der nachfolgend beschriebenen Erfindung die Idee der Fluoreszenzdiagnose bei pseudosimultaner Weißlichtdiagnose verwirklicht. Hierfür ist ein Chopperrad vorgesehen, bei dem ein Kreissegment ein Fluoreszenzanregungsfilter enthält. Dieses sorgt dafür, dass für einen Bruchteil der Umdrehungszeit des Chopperrades das Gewebe mit dem entsprechend gefilterten Licht angeregt wird. Im gleichen Zeitabschnitt ― und nur in diesem - wird auch das vom Gewebe abgegebene Signal einem Fluoreszenzdetektor zugeführt. Ein Bandpassfilter im Chopperrad sorgt dafür, dass das vom Gewebe remittierte Anregungslicht herausgefiltert wird und nur das Fluoreszenzlicht passieren kann. Während eines weiteren Bruchteils der Umdrehungszeit des Chopperrades erfolgt die Beleuchtung und Beobachtung des Gewebes unter ungefiltertem Beleuchtungslicht (Weißlicht), d.h., es befindet sich kein Fluoreszenzanregungsfilter im Beleuchtungsstrahlengang. Der Eingang des Fluoreszenzdetektors ist nun durch das Chopperrad blockiert und erhält dementsprechend kein Licht. Dieses Vorgehen mit Chopperrad und damit realisiertem "Time-Sharing-Verfahren" erlaubt eine Fluoreszenzdiagnose bildgebender oder spektralanalytischer Art bei pseudosimultaner Beobachtung des zu untersuchenden Gewebes unter ungefiltertem Beleuchtungslicht (Weißlicht) als Orientierungshilfe bzw. für die Lokalisierung des mit dem Fluoreszenzverfahren untersuchten Gewebebereichs.

Aber auch diese Vorrichtung ist für eine punktuelle Fiuoreszenzspektroskopie mit hoher Ortsauflösung und mit Sichtbarmachung, d.h. optischer Abhebung desjenigen Gewebebereichs, der die Signale für das Spektrometer liefert, bei pseudosimultaner großflächiger Umgebungsbeleuchtung als Orientierungshilfe und Lokalisierung des punktuell spektroskopisch untersuchten Gewebebereichs ungeeignet. Wie in der DE 196 18 963 C2 besteht auch bei dieser Vorrichtung die Problematik darin, dass unter der Voraussetzung, dass aus Gründen der Kompaktheit und der bequemen Handhabbarkeit nur ein Lichtprojektor mit einem Lichtanschluß verwendet werden soll, nur eine Faser / Faserbündel verwendet wird, welche / welches für die Beleuchtung und für die Fluoreszenzanregung dienen muss. Der Fluoreszenzanregungslichtkegel und der Beleuchtungslichtkegel sind demnach gleich groß. Soll das Gewebe großflächig eingesehen werden können und aber dennoch eine punktuelle Fluoreszenzspektroskopie durchgeführt werden, muss die Einstellung der örtlichen Auflösung, d.h. die Einstellung der Größe des detektierten Gewebebereichs, über die *Detektions*faser / *Detektions*faserbündel vorgenommen werden, z.B. in der Art, wie in DE 196 18 963 C2 beschrieben. Dementsprechend können aber Ort und Durchmesser des detektierten Gewebebereichs für den untersuchenden Arzt vom größerflächigen, die suspekte Stelle umgebenden und mit Beleuchtungslicht angestrahlten Gewebe optisch nicht abgehoben und damit nicht sichtbar gemacht werden.

Das aus "Bronchoscopy" bekannte System wurde auch für die medikamenteninduzierte Fluoreszenzdiagnose entwickelt, bei der - wie bereits erwähnt - bei entsprechender Vorgehensweise auf die Sichtbarmachung des detektierten Gewebebereichs verzichtet werden kann, da bei der medikamenteninduzierten Fluoreszenz ohnehin nur krankes Gewebe fluoresziert.

Demgemäß liegt der vorliegenden Erfindung die Aufgabe zugrunde, eine Diagnosevorrichtung zu schaffen, die die vorgenannten Nachteile überwindet.

Weiterhin soll eine schnelle und einfache Umschaltung zwischen den einzelnen Modi möglich sein. Die Vorrichtung soll also kompakt ausgebildet sein, und die drei Diagnosemethoden sollen mit nur einem Lichtprojektor, welcher zwei Leuchtmittel enthält, mit einer Lichtbuchse, d.h. einem Lichtleiterabgang, durchgeführt werden können.

Diese Aufgabe wird durch die im Anspruch 1 angegebene Vorrichtung gelöst. Weitere vorteilhafte Merkmale der Vorrichtung sind in den Unteransprüchen angegeben.

Die Vorrichtung enthält zwei Leuchtmittel. Der Strahlengang des zweiten Leuchtmittels wird vor dem Verlassen der Vorrichtung dem Strahlengang des ersten Leuchtmitteis überlagert. Die Lichtstrahlenbündel beider Strahlengänge werden dann in einen an ein Endoskop angeschlossenen Lichtleiter eingekoppelt. Im ersten Strahlengang sind Elemente angeordnet, welche bewirken, dass das Licht des ersten Leuchtmittels mit einer relativ großen Bündelöffnung in den Lichtleiter geleitet wird. Im zweiten Strahlengang sind Elemente angeordnet, welche bewirken, dass das Licht des zweiten Leuchtmittels mit einer vergleichsweisen kleinen Bündelöffnung in den Lichtleiter eingeleitet wird.

Im ersten Strahlengang sind Mittel angeordnet, welche das Lichtstrahlenbündel des ersten Strahlengangs zeitweise freigeben bzw. unterbrechen.

Die Führung des Lichts des zweiten Strahlengangs zum Lichtleiter erfolgt mittels Spiegeln, von denen mindestens einer teildurchlässig sein muß. Hinter dem teildurchlässigen Spiegel kann ein Spektrometer angeordnet sein. Dabei ist mit Vorteil vorgesehen, dass vor dem Spektrometer Mittel angeordnet sind, mit denen das Lichtstrahlenbündel, welches vom untersuchten Gewebe zum Spektrometer geleitet wird, zeitweise freigegeben bzw. unterbrochen werden kann. Diese Mittel können auch so angeordnet sein, daß sie gleichzeitig das Lichtstrahlenbündel aus dem zweiten Strahlengang vom zweiten Leuchtmittel zum Lichtleiter freigeben bzw. unterbrechen können.

Die Mittel zum zeitweisen Freigeben und Unterbrechen der Lichtstrahlenbündel bestehen aus einem ersten und einem zweiten Chopperrad. Diese können jeweils eine opake Scheibe aufweisen, die jeweils über einen definierten Winkelbereich eine Ausnehmung haben.

Die Winkelbereiche der Ausnehmungen der beiden synchron antreibbaren Chopperräder sind zueinander komplementär so ausgebildet, dass dem ausgenommenen Bereich des ersten Chopperrades ein abdeckender bzw. opaker Bereich des zweiten Chopperrades entspricht und dass dem abdeckenden bzw. opaken Bereich des ersten Chopperrades ein ausgenommener Bereich des zweiten Chopperrades entspricht.

Als Element für die Bündelöffnungsweitung am Ort der Einkopplung des Lichts des ersten Strahlengangs in den Lichtleiter kommt ein vergleichsweise stark fokussierendes optisches Element wie z.B. eine kurzbrennweitige Linse in Frage. Die gleiche Wirkung hinsichtlich der Bündelöffnung kann aber auch mit einer Schrägeinkopplung des Lichtstrahlenbündels aus dem ersten Strahlengang relativ zur optischen Achse des Lichtleiters erreicht werden oder aus einer Kombination aus beiden Maßnahmen.

Als Element für die Bündelöffnungsbegrenzung am Ort der Einkopplung des Lichts des zweiten Strahlengangs in den Lichtleiter kommen ein Teleskop mit entsprechendem Brennweitenverhältnis seiner beiden Linsen und / oder eine aperturbegrenzende Blende im kolliminierten zweiten Strahlengang in Frage, wobei die aperturbegrenzende Blende durch die begrenzte Ausdehnung oder Fassung eines oder mehrerer optischer Elemente im zweiten Strahlengang bestehen kann. Die gleiche Wirkung hinsichtlich der Bündelöffnung kann aber auch dadurch erzielt werden, dass als zweites Leuchtmittel eine Lichtquelle verwendet wird, welche ein kollimiertes Lichtstrahlenbündel mit kleinem Durchmesser emittiert.

Als Lichtleiter kann ein Fluid-Lichtleiter oder ein bis zum distalen Ende des Endoskops durchgehendes Faserbündel oder eine bis zum distalen Ende des Endoskops durchgehende Einzelfaser vorgesehen werden, wobei idealerweise Lichtleiter mit einer hohen Transmission im Fluoreszenzanregungsband zu verwenden sind.

Das zweite Leuchtmittel, weiches das Licht des zweiten Strahlengangs bereitstellt, ist idealerweise ein kompakter Laser, z.B. ein Diodenlaser, idealerweise mit einem kollimierten Lichtstrahlenbündel von geringem Durchmesser und einem Emissionsbereich, welcher im Fluoreszenzanregungsband des zu untersuchenden Gewebes liegt. Denkbar ist jedoch auch eine Leuchtdiode oder ein Leuchtdiodenarray mit einem entsprechenden Emissionsbereich und vorgelagerter strahlformender Optik oder eine Mischgaslampe, eine Kurzbogenlampe oder eine Glühlampe mit vorgelagertem optischen Bandpaßfilter und strahlformender Optik.

Die punktuelle Fluoreszenzanregung, die Fluoreszenzdetektion und die Beleuchtung des die verdächtige Stelle umgebenden gesunden Gewebes mit Weißlicht erfolgen im Arbeitsmodus C der punktuellen Fluoreszenzspektroskopie über denselben Lichtleiter, nämlich jenen, der auch in den beiden anderen Arbeitsmodi A und B für die Beleuchtung bzw. Anregung verwendet wird. Das bedeutet, dass keine zusätzlichen Fasern bzw. Faserbündel für die punktuelle Fluoreszenzspektroskopie erforderlich sind. Der Instrumentenkanal des Endoskops bleibt beispielsweise für Probeentnahmen permanent frei, und die Handhabung des Systems kann auf einfache Art und Weise erfolgen.

Für den Aufbau des Endoskops ergibt sich ein einfaches Konzept, da nur ein Lichtleiter, nämlich der in den Arbeitsmodi A und B zur Beleuchtung und Anregung verwendete, sowohl für die Beleuchtung bzw. Anregung als auch für die Spektrometerdetektion erforderlich ist. Es reicht also die gewöhnliche Optik eines Endoskops aus. Der apparative Aufbau ist also im Sinne der Aufgabenstellung einfach gehalten.

Eine Änderung des Durchmessers des sogenannten zentralen Spektraldetektionsfeldes, also des Durchmessers jenes Gewebebereichs, dessen Fluoreszenz dem Spektrometer zugeführt wird, kann einfach durch Verstellen eines Elements, wie beispielsweise einer Iris-Blende oder eines mehrere Blenden mit unterschiedlichem Durchmesser aufweisendes Blendenrad oder dergleichen, in der Lichtquelle im Anregungskanal für die Fluoreszenzspektroskopie, d.h. im zweiten Strahlengang, bewerkstelligt werden.

Weiterhin erlaubt ein schnelles und einfaches Hin- und Herschalten zwischen den Diagnosemethoden der einzelnen Modi A, B und C das Auffinden der verdächtigen Stellen im konventionellen Weißlichtmodus (Modus A) oder im großflächigen bildgebenden Fluoreszenzdiagnosemodus (Modus B) und ein anschließendes schnelles Umschalten in den Modus der punktuellen Fluoreszenzspektroskopie (Modus C). Hierdurch wird mit der sogenannten optischen Biopsie eine detaillierte Bewertung anhand des Fluoreszenzspektrums möglich.

Es wird mit der erfindungsgemäßen Vorrichtung möglich, alle drei genannten Diagnoseverfahren mit einem Lichtprojektor mit einer Lichtbuchse, d.h. einem Lichtleiterabgang, und damit mit einer optischen Verbindung von Lichtquelle und Endoskop durchzuführen.

Dazu wird mit Bündelöffnungen unterschiedlicher Größe in den für alle Diagnosemodi gleichen Lichtleiter eingekoppelt, und zwar das Weißlicht in den Modi A und C und das Anregungslicht im Modus B aus dem ersten Strahlengang mit einer großen Bündelöffnung am Ort der Einkopplung in den Lichtleiter und das Anregungslicht im Modus C für die punktuelle Anregung aus dem zweiten Strahlengang mit einer kleinen Bündelöffnung am Ort der Einkopplung in den Lichtleiter - die proximalseitige Einkopplung von Licht mit einer großen Bündelöffnung bewirkt beim beschriebenen Aufbau distal eine großflächige Ausleuchtung bzw. Fluoreszenzanregung; eine proximalseitige Einkopplung von Licht mit einer kleinen Bündelöffnung hingegen bringt beim erläuterten Aufbau distal eine kleinflächige oder gar punktuelle Ausleuchtung bzw. Fluoreszenzanregung. Dadurch wird es im Modus C möglich, eine großflächige weiße Hintergrundbeleuchtung zur Orientierung für den untersuchenden Arzt zu erzeugen (großer Beleuchtungslichtkegel am distalen Ende der Beleuchtungs- bzw. Anregungsfaser des Endoskops) und den Gewebebereich, der das dem Spektrometer zugeführte Fluoreszenzlicht liefert, als entsprechend kleinen und im Falle der Autofluoreszenzanregung blauen Fleck dem Weißlicht zu überlagern (kleiner bzw. schlanker Anregungslichtkegel am distalen Ende der Beleuchtungs- bzw. Anregungsfaser des Endoskops) und damit Ort und Durchmesser des spektrometrisch untersuchten Bereichs dem Anwender direkt sichtbar zu machen.

Die vorgeschlagene Vorrichtung zeichnet sich auch durch einen sehr kompakten Aufbau aus. Alle drei Untersuchungsmodi können nämlich mit nur einem Lichtprojektor durchgeführt werden. Weiterhin ermöglicht die Vorrichtung im Diagnosemodus der punktuellen Fluoreszenzspektroskopie die Untersuchung an einem konventionellen großflächigen Weißlichtbild. Der Ort und der Durchmesser bzw. die Abmessungen des punktuell spektroskopisch untersuchten verdächtigen Gewebebereiches lassen sich dabei klar erkennen und unterscheiden vom lediglich zur besseren Orientierung und Lokalisierung weiß beleuchteten umgebenden Gewebe. Der punktuell spektroskopisch untersuchte verdächtige Gewebebereich, also derjenige, dessen Fluoreszenzlicht für die Erzeugung der Spektrometerkurve herangezogen wird, hebt sich deutlich als blauer Fleck vom großflächig umgebenden, weißes Beleuchtungslicht remittierenden und nicht verdächtigen Gewebe ab.

Es besteht vor allem die Möglichkeit einer einfachen Durchmesserveränderung des punktuell spektroskopisch untersuchten Gewebebereichs und damit die Möglichkeit der Größenanpassung der spektroskopisch detektierten Stelle an die Größe der verdächtigen Stelle. Dabei ist dann die Durchmesserveränderung, also die veränderte Größe des spektroskopisch untersuchten Gewebebereichs, direkt sichtbar, nämlich als die Größenänderung des "blauen Anregungslichtflecks" vor dem Hintergrund des weiß beleuchteten Gewebes.

Schließlich ergibt sich eine einfache Handhabung der Vorrichtung beim Wechsel zwischen den einzelnen Diagnosemodi. Der Wechsel zwischen den unterschiedlichen Diagnosemethoden erfordert lediglich die Betätigung eines Schalters, einer Taste oder dergleichen. Es ist nicht notwendig, Lichtleiter umzustecken, eine Zusatzvorrichtung auf das Endoskop zu setzen oder gar das Endoskop zu wechseln.

In der Zeichnung ist ein Ausführungsbeispiel der Erfindung schematisch dargestellt. Es zeigen:
- Fig. 1: den Aufbau einer Diagnosevorrichtung,
- Fig. 2: die Draufsicht auf die Scheibe eines ersten Chopperrades,
- Fig. 3: die Draufsicht auf die Scheibe eines zweiten Chopperrades und
- Fig. 4: die Draufsicht auf eine Scheibe mit unterschiedliche Durchmesser aufweisenden Blenden.

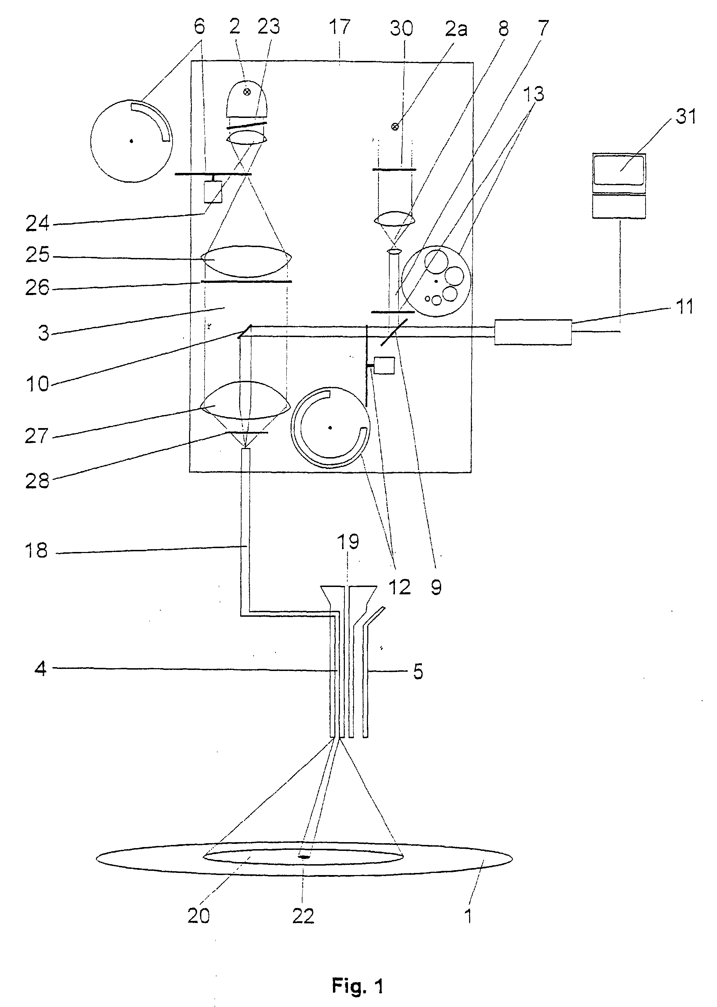

Die Diagnosevorrichtung in Fig. 1 besteht aus einem Lichtprojektor 17, der durch das eingezeichnete Rechteck umrandet ist. Der Lichtprojektor 17 hat ein erstes Leuchtmittel 2, weiches das fokussierte Licht in einen ersten Strahlengang 3 abgibt. Über einen Lichtleiter 18 gelangt das Licht aus dem ersten Strahlengang 3 in die Beleuchtungs- bzw. Anregungsfaser 4 eines Endoskops 5.

Das distale Ende des Endoskops 5 ist auf das zu untersuchende Gewebe 1 gerichtet. Über das nicht näher dargestellte Okular 19 kann das Gewebe 1 betrachtet werden. Die Optik im ersten Strahlengang, beispielsweise die vergleichsweise kurzbrennweitige Linse 27, sowie die weitere Lichtleitung über den Lichtleiter 18 und die Beleuchtungs- bzw. Anregungsfaser 4 bewirken, dass das vom ersten Leuchtmittel 2 über den ersten Strahlengang 3 bis zum Gewebe 1 geleitete Licht dort einen relativ großen Bereich 20 ausleuchten. Der untersuchende Arzt kann über das Okular 19 einen relativ großen Gewebebereich 20 über das vom ersten Leuchtmittel 2 stammende Weißlicht einsehen und großflächig beurteilen.

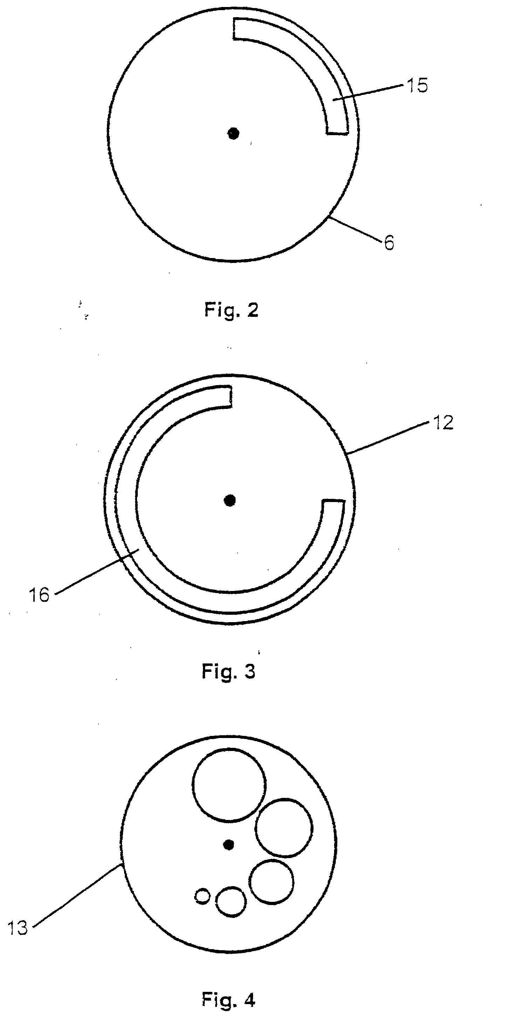

In diesem Untersuchungsmodus A der bildgebenden Weißlichtdiagnose liefert das Leuchtmittel 2 permanent und ununterbrochen Licht an das Gewebe 1. Ein sich im ersten Strahlengang 3 befindliches erstes Chopperrad 6 ist so ausgebildet, dass es steuerbar das Lichtstrahlenbündel vom Leuchtmittel 2 bis zum Gewebe 1 entweder blockiert oder freigibt. Daher ist das Chopperrad im Modus A stillstehend so gedreht bzw. positioniert, dass eine Ausnehmung 15 in der Chopperrad-Scheibe 14 (Fig. 2) permanenten Lichtdurchgang sicherstellt.

In der Lichtquelle 17 befindet sich ein zweites Leuchtmittel 2a, weiches ein kollimiertes Lichtstrahlenbündel in einen zweiten Strahlengang 7 abgibt. Dieser verläuft zunächst parallel zum ersten Strahlengang 3. Hier kann ein aus zwei Elementen bestehendes Linsensystem 8 (Teleskop) angeordnet sein, wobei anstelle des Teleskops 8 ein beliebiges anderes den Strahldurchmesser reduzierendes Element, beispielsweise eine aperturbegrenzende Blende, vorgesehen werden kann. Die aperturbegrenzende Wirkung kann auch durch eine entsprechend begrenzte Ausdehnung von wenigstens einem der Spiegel oder eines anderen sich im Strahlengang befindlichen Element erzielt werden. Wesentlich ist lediglich, dass die Größe des Durchmessers des parallelen Lichtstrahlenbündels im zweiten Strahlengang 7 an der Stelle, wo es die Linse 27 transmittiert, relativ gering ist. Dadurch wird gewährleistet, dass das das Endoskop 5 verlassende Lichtstrahlenbündel, welches aus dem zweiten Strahlengang 7 stammt, am Gewebe mit einem vergleichsweise geringen Durchmesser 22 auftrifft. Will man die Größe des Durchmessers des kollimierten Lichtstrahlenbündels im zweiten Strahlengang 7 und damit die Größe des das Endoskop 5 verlassenden Anregungslichtkegels verstellbar gestalten, so kann dies beispielsweise über eine Iris-Blende oder über ein verstellbares mehrere Blenden mit unterschiedlichen Durchmessern aufweisendes Blendenrad 13 erfolgen.

Das im Durchmesser verringerte Lichtstrahlenbündel des zweiten Strahlengangs 7 wird über einen teildurchlässigen Spiegel 9 und einen Spiegel 10 dem ersten Strahlengang 3 überlagert und durch den Lichtleiter 18 dem Endoskop 5 zugeleitet. Über die Beleuchtungs- und Anregungsfaser 4 gelangt das Licht auf das Gewebe 1, jedoch aufgrund des reduzierten Durchmessers des kollimierten Lichtstrahlenbündels am Ort der Transmission von Linse 27 und damit aufgrund der verringerten Bündelöffnung des Lichtstrahlenbündels aus dem zweiten Strahlengang 7 am Ort der Einkopplung in den Lichtleiter 18 nur auf einen wesentlich kleineren Bereich 22 als das Licht aus dem ersten Strahlengang, welches den größeren Gewebebereich 20 ausleuchtet. Der teildurchlässige Spiegel 9 ist so ausgeführt, dass er Anregungslicht reflektiert und Fluoreszenzlicht transmittiert.

Hinter dem teildurchlässigen Spiegel 9 befindet sich in zumindest zeitweise freigebender optischer Verbindung ein Spektrometer 11, in das vom Gewebebereich 22 abgegebenes Fluoreszenzlicht zwecks Spektralanalyse gelangen kann. Auf einem Monitor 31 kann das Ergebnis der Spektralanalyse als Spektrometerkurve dargestellt werden.

Zur punktuellen Fluoreszenzspektroskopie (Modus C) muß sichergestellt sein, dass zum Spektrometer 11 nur und ausschließlich vom Gewebe 1 aus dem kleinen angeregten Bereich 22 stammendes Fluoreszenzlicht gelangt, nicht dagegen Weißlicht des ersten Leuchtmittels 2, welches vom Gewebe remittiert wird. Hierzu ist ein zweites Chopperrad 12 vor dem Spektrometer 11 angeordnet, das analog dem Chopperrad 6 aufgebaut ist. Die Scheibe dieses Chopperrades 12 (Fig. 3) weist eine über einen definierten Umfangsbereich reichende Ausnehmung 16 auf, die insofern komplementär zu der Ausnehmung 15 der Scheibe des ersten Chopperrades 6 ist, als dass bei simultaner Drehung beider Chopperräder 6 und 12 niemals Licht vom ersten Leuchtmittel 2, weiches über den ersten Strahlengang 3 an das zu untersuchende Gewebe gelangt und von dort remittiert wird und über die Faser 4, den Lichtleiter 18 sowie den Spiegel 10, der nicht teildurchlässig ist, an den Ort des Chopperrads 12 gelangt, in das Spektrometer 11 gelangen kann. Durch die Positionierung des Chopperrades 12 vor dem teildurchlässigen Spiegel 9 erreicht man, dass beispielsweise im Modus A, wenn das Spektrometer geblockt ist, auch kein durch das Leuchtmittel 2a erzeugter blauer Fleck der weißen Ausleuchtung des Objektfeldes überlagert wird.

In Fig. 1 sind neben den Chopperrädern 6 und 12 schematisch deren Scheiben in Draufsichten dargestellt, aus denen sich das stellungsmäßige Zusammenwirken der Ausnehmungen 15 und 16 entnehmen läßt. Entsprechend ist auch die Draufsicht auf ein mehrere Blenden mit unterschiedlichen Durchmessern aufweisendes Blendenrad 13 skizziert (s. auch Fig. 4)

In Fig. 1 sind noch verschiedene Details eingezeichnet, die die Lichtquelle 17 aufweist. Das erste Leuchtmittel 2 ist eine Weißlichtquelle, wobei bevorzugt eine Bogenlampe mit Spiegelreflektor (Paraboloid oder Ellipsoid) eingesetzt wird. Denkbar ist jedoch auch ein Kondensorsystem. Als Alternative kommt auch eine Glühwendellampe (z.B. eine Halogenlampe) in Frage. Ein Filter 23 wirkt als Bandpaß, der IR- und UV-Strahlung herausfiltert. Zum Teil erfolgt dies aber auch schon durch die Reflektorbeschichtung des Leuchtmittels 2. Die Linse 24 erzeugt einen ersten Fokus, in welchem sich idealerweise aber nicht notwendigerweise das Chopperrad 6 befindet. Wird ein elliptischer Reflektor verwendet, kann auf die Linse 24 verzichtet werden, und das Chopperrad 6 wird idealerweise im zweiten Brennpunkt des Ellipsoids positioniert.

Das Chopperrad 6 befindet sich bei der konventionellen Weißlichtdiagnose (Modus A) und bei der bildgebenden Fluoreszenzdiagnose (Modus B) in Ruhestellung und auf Durchlaß (Ausnehmung 15 im Strahlengang). Die Linse 25 erzeugt ein kollimiertes Strahlenbündel, in dessen Verlauf bei der bildgebenden Fluoreszenzdiagnose ein Filter 26 eingeschwenkt wird, dessen Transmissionseigenschaften auf eine optimale Fluoreszenzanregung des zu untersuchenden biologischen Gewebes 1 abgestimmt sind, wie etwa ein Blaufilter bei der Autofluoreszenzanregung menschlichen Gewebes beispielsweise im Bronchialtrakt oder im Ösophagus.

Das Filter 26 selektiert also aus dem breitbandigen Weißlicht des Leuchtmittels 2 den erforderlichen optimalen Spektralbereich für die Fluoreszenzanregung. Bei der konventionellen Weißlichtdiagnose ist dieses Filter aus dem kollimierten Strahlengang herausgeschwenkt. Die Linse 27 fokussiert das blaue Anregungslicht im Modus B der Fluoreszenzdiagnose bzw. das weiße Beleuchtungslicht im Modus A der konventionellen Weißlichtdiagnose und im Modus C der punktuellen Fluoreszenzspektroskopie so stark, dass am distalen Ende des an die Lichtquelle 17 über den Lichtleiter 18 angeschlossenen Endoskops 5 der Anregungs- bzw. Beleuchtungsstrahlenkegel ausreichend groß ist, ausreichend groß in dem Sinne, dass bei entsprechendem Abstand zwischen Endoskopspitze und Gewebe ein genügend großer Gewebebereich 20 ausgeleuchtet wird und überblickend eingesehen werden kann.

Das Fluoreszenzanregungslicht bei der bildgebenden Fluoreszenzdiagnose (Modus B) bzw. das weiße Beleuchtungslicht bei der konventionellen Weißlichtdiagnose (Modus A) und die weiße Umgebungsbeleuchtung bei der punktuellen Fluoreszenzspektroskopie (Modus C) werden in den Lichtleiter 18 eingekoppelt. Eine nicht aperturbegrenzende Blende 28 erlaubt eine Steuerung der an das Gewebe herangeführten Lichtstrommenge.

im Arbeitsmodus C der punktuellen Fluoreszenzspektroskopie beginnt sich das Chopperrad 6 mit hoher Frequenz, wie beispielsweise mit der Videofrequenz, zu drehen. Das Filter 26 für die Fluoreszenzanregung ist in diesem Betriebsmodus aus dem Strahlengang ausgeschwenkt. Steht das Chopperrad 6 in der Stellung "Durchlass", erreicht den Lichtleiter 18 für den der Größe des offenen Kreissegments 15 entsprechenden Bruchteil der Umdrehungsdauer Weißlicht, für den verbleibenden Bruchteil der Umdrehungsdauer, also während das Chopperrad 6 blockierend im Strahlengang steht, erreicht kein Weißlicht den Lichtleiter 18 und damit das zu untersuchende Gewebe 1. Stattdessen wird nun Licht des Leuchtmittels 2a über den Strahlengang 7 und über die Spiegel 9 und 10, sowie über die Linse 27 in den Lichtleiter 18 eingekoppelt.

Ein Filter 30, das sich permanent im Strahlengang 7 befindet, selektiert aus dem Licht des Leuchtmittels 2a das für die Fluoreszenzspektroskopie ideale Anregungslicht. Handelt es sich bei 2a bereits um ein Leuchtmittel mit einem spektral relativ schmalen Emissionsband, wie z.B. einem Laser, und liegt dieses Emissionsband voll im Fluoreszenzanranregungsband, dann kann auf das Filter 30 verzichtet werden.

Die Fluoreszenzspektroskopie soll, wie oben eingehend erläutert, mit Vorteil punktuell erfolgen, d.h. ein möglichst kleiner Anregungslichtkegel 22 am distalen Ende der Beleuchtungs- bzw. Anregungsfaser 4 soll eine bezüglich des Ortes entsprechend hochaufgelöste Fluoreszenzspektroskopie und damit die Aufnahme und Beurteilung von entsprechend kleinen Läsionen erlauben. Dementsprechend gering muß der Durchmesser des kollimierten Strahlenbündels aus dem Strahlengang 7 am Ort der Linse 27 sein, d.h. dementsprechend hoch muß die Reduktion des Durchmessers des kollimierten Strahlenbündels des Leuchtmittels 2a sein. Dies erfolgt über die Wahl des Brennweitenverhältnisses der Linsen des Teleskops 8: Je größer der Quotient aus der Brennweite der dem Leuchtmittel 2a zugewandten Linse durch die Brennweite der dem Leuchtmittel 2a abgewandten Linse des Teleskops 8, um so stärker ist die Reduktion des Durchmessers des vom Leuchtmittel 2a emittierten kollimierten Strahlenbündels. Gleichermaßen den Strahldurchmesser reduzierend wirkt beispielsweise der begrenzte Durchmesser des Spiegels 10. Besteht das Leuchtmittel 2a beispielsweise aus einem Laser, welcher einen kollimierten Strahl mit entsprechend geringem Durchmesser emittiert, kann u.U. auf weitere, den Strahldurchmesser reduzierende Maßnahmen verzichtet werden. Eine weitere, nicht dargestellte und optisch dämpfend wirkende Einrichtung, wie beispielsweise ein Neutralfilter, kann eine Regulierung der Intensität des die Fluoreszenz erzeugenden Anregungslichts ermöglichen.

In einer weiteren Ausführungsform ist ein verstellbares, mehrere Blenden mit unterschiedlichen Durchmessern aufweisendes Blendenrad 13 (Fig. 4) in den Strahlengang eingebracht, wobei auch andere aperturverstellbare Einrichtungen, wie z.B. eine Irisblende, denkbar sind. In dieser Ausführungsform kann das Brennweitenverhältnis der Linsen des Teleskops 8 nahe bei Eins liegen oder es kann auf das Teleskop 8 ganz verzichtet werden.

Durch Verdrehen des Blendenrades 13 sind der Anregungslichtkegel und damit das (örtliche) Auflösungsvermögen bei der Fluoreszenzspektroskopie nahezu beliebig verstellbar. Ist die suspekte Stelle großflächig, kann eine große Blende im Blendenrad 13 gewählt werden, um nahezu die gesamte verdächtige Gewebefläche anzuregen. Falls ein höheres Auflösungsvermögen gefordert ist, weil der suspekte Gewebebereich nur einen relativ kleinen Durchmesser hat, kann eine kleine Blende im Blendenrad 13 gewählt werden. Damit ist gewährleistet, dass der Verlauf der ermittelten Spektralkurven im Falle einer Autofluoreszenzdiagnose nicht durch die Fluoreszenz von dem der verdächtigen Stelle benachbarten Gewebe bestimmt bzw. mitbestimmt wird.

Synchron, d.h. mit der gleichen (vergleichsweisen hohen) Drehfrequenz und in einer festgelegten Phase zur Bewegung des Chopperrades 6 dreht sich im Diagnosemodus C der punktuellen Fluoreszenzspektroskopie zwischen den Spiegeln 9 und 10 das zweite Chopperrad 12. Die Größe der Ausnehmungen 15 und 16 (Fig. 2 und 3) ist nur ein Beispiel für deren Ausgestaltung. Ist jedoch bei einem Chopperrad 6 die Ausnehmung 15 festgelegt, ergibt sich die andere Ausnehmung 16 am anderen Chopperrad 12 automatisch. Während der Beleuchtung des Gewebes mit Weißlicht, das erste Chopperrad 6 befindet sich also gerade für diesen Augenblick auf Durchlass, ist der Eingang zum Spektrometer 11 verdeckt; außerdem kann Fluoreszenzanregungslicht des Leuchtmittels 2a das Gewebe 1 nicht erreichen. in der Anregungsphase des Gewebes hingegen, das Chopperrad 6 sperrt nun, ist der Eingang des Spektrometers 11 unverdeckt. Durch die hohe Drehfrequenz der beiden Chopperräder, beispielsweise Videofrequenz, erscheint die punktuelle Fluoreszenzanregung und die Beleuchtung des die verdächtige Stelle umgebenden Gewebes mit Weißlicht quasi simultan. Desweiteren kann vor dem Spektrometer 11 ein Filter angeordnet sein (in Fig. 1 nicht eingezeichnet), welches nur das Fluoreszenzlicht transittiert, das Licht jenseits dieses Spektralbereichs jedoch blockt. Diese Aufgabe kann aber auch bereits von Spiegel 9 übernommen werden, wenn dieser als entsprechender Interferenzfilter mit entsprechend strengen Spezifikationen ausgeführt ist. Das Spektrometer 11 kann also nur Fluoreszenzlicht empfangen, niemals aber vom Gewebe remittiertes weißes Beleuchtungs- oder Anregungslicht.

Das System enthält eine zentrale Steuereinheit, die bei Umschaltvorgängen zwischen den einzelnen Untersuchungsmodi folgende Abläufe in der Lichtquelle 17 und am Spektrometer 11 koordiniert. Wenn die Vorrichtung in den Modus A der konventionellen und daher großflächigen Weißlichtdiagnose geschaltet wird, wird das Chopperrad 6 abgebremst (sofern es vorher in Drehbewegung war) und bleibt während dieses Arbeitsmodus' in Ruhestellung, und zwar in Durchlaßstellung bezüglich des Lichtes des ersten Strahlengangs 3. Gleichzeitig wird dafür gesorgt, dass das Filter 26 ausgeschwenkt ist. Das gesamte Weißlicht wird in den Lichtleiter 18 eingekoppelt. Das zweite Chopperrad 12, das zwischen den Spiegeln 9 und 10 positioniert ist, wird, sofern es vorher in Drehbewegung war, ebenfalls abgebremst und bleibt während der gesamten Zeit in diesem Arbeitsmodus sperrend in Ruhestellung. Dadurch wird einerseits verhindert, dass vom Gewebe remittiertes Beleuchtungslicht in das Spektrometer gelangt und andererseits vermieden, dass auf dem Gewebe ein im Modus A unerwünschter blauer Fleck vom Leuchtmittel 2a auf dem Gewebe erscheint.

Beim Umschalten in den Modus B der bildgebenden Fluoreszenzdiagnose bleiben die Stellungen der Chopperräder unverändert, d.h., das Chopperrad 6 bleibt in Ruhe und auf Durchlass, und das Chopperrad 12 bleibt ebenfalls in Ruhe und sperrend. Gleichzeitig wird das Filter 26 in den Strahlengang 3 eingeschwenkt.

Wird in den Arbeitsmodus C der punktuellen Fluoreszenzspektroskopie geschaltet, sorgt die zentrale Steuereinheit dafür, dass das Filter 26 aus dem Strahlengang 3 ausgeschwenkt wird, wodurch Weißlicht in den Lichtleiter 18 eingekoppelt werden kann. Beide Chopperräder 6 und 12 fangen an, sich mit hoher Frequenz, wie beispieisweise mit der Videofrequenz, zu drehen, und zwar in der Art, dass in der Durchlaßstellung des Chopperrades 6 das Chopperrad 12, das zwischen den Spiegeln 9 und 10 positioniert ist, sperrt. In der Phase der Weißlichtbeleuchtung des Gewebes 1 empfängt also das Spektrometer kein Licht und das Gewebe 1 wird auch nicht punktuell angeregt mit Licht aus dem Strahlengang 7. in der Sperrstellung des Chopperrades 6 ist das Chopperrad 12 zwischen den Spiegeln 9 und 10 transmittierend, d.h. das Gewebe 1 wird punktuell mit über das Filter 30 (sofern die Art des Leuchtmittels 2a dies erfordert) gefiltertem Licht aus der Lichtquelle 2a, welches über die Spiegel 9 und 10 sowie über die Linse 27 in den Lichtleiter 18 eingekoppelt wird, angeregt und das Spektrometer 11 kann Fluoreszenzlicht, welches über das Endoskop 5, den Lichtleiter 18 und den Spiegel 10 geleitet wird und den halbdurchlässigen Spiegel 9 transmittiert, empfangen.

Während in Fig. 1 dargestellt ist, wie der Durchmesser des Lichtstrahlenbündels im zweiten Strahlengang 7 mittels eines Teleskops 8 auf den gewünschten kleineren Wert reduziert wird, kann diese Durchmesserreduktion auch in beliebiger anderer Weise mittels eines geeigneten Bündelungs- oder Begrenzungselements erfolgen.

Eine diesbezüglich besonders einfache Ausbildung ergibt sich, wenn vorgesehen wird, dass unter Verzicht auf das Teleskop 8 ein im Durchmesser entsprechend klein gehaltener Spiegel 10 zum Einsatz kommt. Wegen der Schrägstellung von 45 Grad wird dann ein elliptischer Spiegel 10 eingesetzt, so dass seine Projektionsfläche in Richtung der optischen Achse des ersten Strahlengangs 3 kreisrund wird. Dabei ergibt sich auch der Vorteil, dass im Modus A bzw. im Modus 8 nur ein geringer Teil des Lichts vom ersten Leuchtmittel 2 an der Rückseite des bevorzugt fest im Strahlengang 3 montierten Spiegels 10 blockiert wird. Eine Lichteinbuße in den Modi A und B wird damit nahezu vollständig verhindert. Es kann sonst auch vorgesehen werden, dass der Spiegel 10 aus dem Strahlengang 3 herausklappbar ausgeführt ist, um in den Diagnosemodi A und B keine Lichteinbuße zu haben.

Der Spiegel 9 ist so konzipiert, dass er nur für das die Fluoreszenz anregende Licht, z.B. Blaulicht bei der Autofluoreszenzdiagnose, hochreflektierend ist. Er ist jedoch andererseits so ausgeführt, dass er für das Fluoreszenzlicht selbst hochtransmittierend wirkt. Das Fluoreszenzlicht wird hinter dem Spiegel 9 entweder direkt oder über eine weitere sich im Lichtprojektor befindliche und damit das Handling des Systems nicht störende Faser / Faserbündel in das Spektrometer 11 eingekoppelt.

Das Spektrometer kann, wie in Fig: 1 abgebildet, außerhalb der Lichtquelle positioniert sein oder - dadurch wird das Gesamtsystem noch kompakter - im Lichtquellengehäuse untergebracht sein.

## Patentansprüche

1. Vorrichtung zur bildgebenden und spektroskopischen Diagnose von Gewebe (1) unter alternativer oder kombinierter Anwendung von drei Diagnosemethoden, nämlich einem Modus A zur bildgebenden Weißlichtdiagnose, einem Modus B zur bildgebenden Fluoreszenzdiagnose und einem Modus C zur fluoreszenzspektroskopischen Diagnose, wobei die Vorrichtung ein Leuchtmittel (2) aufweist, dessen Licht als Strahlenbündel über einen Strahlengang (3) in einen zu einem Endoskop (5) führenden Lichtleiter (18) eingekoppelt wird, **dadurch gekennzeichnet, dass** die Vorrichtung außer dem vorerwähnten ersten Leuchtmittel (2) ein zweites Leuchtmittel (2a) aufweist, dessen Licht als Strahlenbündel über einen weiteren zweiten Strahlengang (7) vor dem Verlassen der Vorrichtung dem anderen ersten Strahlengang (3) überlagert wird und dann in den Lichtleiter (18) eingekoppelt wird, dass im ersten Strahlengang (3) ein die Bündelöffnung weitendes Element für das in den Lichtleiter (18) eintretende Lichtstrahlenbündel des ersten Strahlengangs angeordnet ist und dass im zweiten Strahlengang (7) ein die Bündelöffnung begrenzendes Element für das in den Lichtleiter eintretende Lichtstrahlenbündel des zweiten Strahlengangs angeordnet ist.

2. Vorrichtung nach Anspruch 1, **dadurch gekennzeichnet, dass** im ersten Strahlengang (3) Mittel (6) angeordnet sind, mit denen das Lichtstrahlenbündel zeitweise freigegeben bzw. unterbrochen werden kann.

3. Vorrichtung nach Anspruch 1, **dadurch gekennzeichnet, dass** die Führung des Lichts des zweiten Strahlengangs (7) zum Lichtleiter (18) mittels eines teildurchlässigen Spiegels (9) erfolgt.

4. Vorrichtung nach Anspruch 3, **dadurch gekennzeichnet, dass** hinter dem Spiegel (9) ein Spektrometer (11) angeordnet ist und dass vor dem Spektrometer (11) Mittel (12) angeordnet sind, mit denen das Lichtstrahlenbündel vor dem Spektrometer (11) zeitweise freigegeben bzw. unterbrochen werden kann.

5. Vorrichtung nach Anspruch 2 und 4, **dadurch gekennzeichnet, dass** die Mittel (6, 12) zum zeitweisen Freigeben und Unterbrechen der Lichtstrahlenbündel aus einem ersten (6) und einem zweiten Chopperrad (12) bestehen, die synchron antreibbar sind, und dass beide Chopperräder (6, 12) opake Flächen aufweisen, die über einen jeweils definierten Bereich die Ausnehmungen (15, 16) aufweisen.

6. Vorrichtung nach Anspruch 5, **dadurch gekennzeichnet, dass** die Ausnehmungen (15, 16) der beiden Chopperräder (6, 12) zueinander komplementär so ausgebildet sind, dass dem ausgenommenen Bereich (15) des ersten Chopperrades (6) ein abdeckender bzw. opaker Bereich des zweiten Chopperrades (12) entspricht und dass dem abdeckenden bzw. opaken Bereich des ersten Chopperrades (6) ein ausgenommener Bereich (16) des zweiten Chopperrades (12) entspricht.

7. Vorrichtung nach einem der Ansprüche 1 bis 6, **dadurch gekennzeichnet, dass** das die Bündelöffnung weitende Element für das in den Lichtleiter (18) eintretende Lichtstrahlenbündel aus dem ersten Strahlengang (3) aus einem stark fokussierenden optischen Element, beispielsweise der kurzbrennweitigen Linse (27), besteht.

8. Vorrichtung nach einem der Ansprüche 1 bis 6, **dadurch gekennzeichnet, dass** das die Bündelöffnung weitende Element für das in den Lichtleiter (18) eintretende Lichtstrahlenbündel aus dem ersten Strahlengang (3) mit einer Schrägeinkopplung des Lichtstrahlenbündels aus dem Strahlengang (3) in den Lichtleiter (18) realisiert wird.

9. Vorrichtung nach einem der Ansprüche 1 bis 6, **dadurch gekennzeichnet, dass** das die Bündelöffnung weitende Element für das in den Lichtleiter (18) eintretende Lichtstrahlenbündel aus dem ersten Strahlengang (3) aus einem Element der Ansprüche 7 und 8 alleine oder aus einer Kombination dieser Elemente besteht.

10. Vorrichtung nach einem der Ansprüche 1 bis 6, **dadurch gekennzeichnet, dass** das die Bündelöffnung begrenzende Element für das in den Lichtleiter (18) eintretende Lichtstrahlenbündel aus dem zweiten Strahlengang (7) aus einem Leuchtmittel (2a), welches ein paralleles Lichtstrahlenbündel mit kleinem Durchmesser emittiert, besteht.

11. Vorrichtung nach einem der Ansprüche 1 bis 6, **dadurch gekennzeichnet, dass** das die Bündelöffnung begrenzende Element für das in den Lichtleiter (18) eintretende Lichtstrahlenbündel aus dem zweiten Strahlengang (7) aus einem Teleskop (8) mit entsprechendem Brennweitenverhältnis seiner beiden Linsen besteht.

12. Vorrichtung nach einem der Ansprüche 1 bis 6, **dadurch gekennzeichnet, dass** das die Bündelöffnung begrenzende Element für das in den Lichtleiter (18) eintretende Lichtstrahlenbündel aus dem zweiten Strahlengang (7) aus einer aperturbegrenzenden Blende im Strahlengang (7) besteht.

13. Vorrichtung nach Anspruch 12, **dadurch gekennzeichnet, dass** die aperturbegrenzende Blende durch die begrenzte Ausdehnung mindestens eines der optischen Elemente im Strahlengang (7) realisiert wird.

14. Vorrichtung nach einem der Ansprüche 1 bis 6, **dadurch gekennzeichnet, dass** das die Bündelöffnung begrenzende Element für das in den Lichtleiter (18) eintretende Lichtstrahlenbündel aus dem zweiten Strahlengang (7) aus einem der Elemente der Ansprüche 10 bis 13 alleine oder aus einer Kombination dieser Elemente besteht.

15. Vorrichtung nach einem der Ansprüche 1 bis 14, **dadurch gekennzeichnet, dass** der dem Endoskop (5) das Licht zuführende Lichtleiter (18) aus einem Fluid-Lichtleiter mit hoher Transmission im Fluoreszenzanregungsband besteht.

16. Vorrichtung nach einem der Ansprüche 1 bis 14, **dadurch gekennzeichnet, dass** der Lichtleiter (18) aus einer Faser oder einem Faserbündel mit hoher Transmission im Fluoreszenzanregungsband besteht.

17. Vorrichtung nach einem der Ansprüche 15 und 16, **dadurch gekennzeichnet, dass** der Lichtleiter (18) bis zum distalen Ende des Endoskops (5) verläuft,

18. Vorrichtung nach einem der Ansprüche 1 bis 6, **dadurch gekennzeichnet, dass** das zweite Leuchtmittel (2a) ein Laser ist.

19. Vorrichtung nach einem der Ansprüche 1 bis 6, **dadurch gekennzeichnet, dass** das zweite Leuchtmittel (2a) eine Mischgaslampe ist.

20. Vorrichtung nach einem der Ansprüche 1 bis 6, **dadurch gekennzeichnet, dass** das zweite Leuchtmittel (2a) eine Leuchtdiode oder ein Array von Leuchtdioden ist.

21. Vorrichtung nach einem der Ansprüche 1 bis 6, **dadurch gekennzeichnet, dass** das zweite Leuchtmittel (2a) eine Kurzbogenlampe ist.

22. Vorrichtung nach einem der Ansprüche 1 bis 6, **dadurch gekennzeichnet, dass** das zweite Leuchtmittel (2a) eine Glühlampe ist.

## Claims

1. A device for the picture-providing and spectroscopic diagnosis of tissue (1) with the alternative or combined use of three diagnosis methods, specifically a mode A for the picture-providing white light diagnosis, a mode B for the picture-providing fluorescence diagnosis and a mode C for the fluorescence-spectroscopic diagnosis, wherein the device comprises an illumination means (2), whose light as a beam bundle via a beam path (3) is coupled into fibre-optic (18) leading to an endoscope (5), **characterised in that** the device apart from the previously mentioned first illumination means (2) comprises a second illumination means (2a) whose light as a beam bundle via a further second beam path (7) before leaving the device is superimposed with the other first beam path (3) and then coupled into the fibre-optic (18), that in the first beam path (3) there is arranged an element widening the bundle opening, for the light beam bundle of the first beam path entering the fibre-optic (18) and that in the second beam path (7) there is arranged an element limiting the bundle opening, for the light beam bundle of the second beam path entering the fibre-optic.

2. A device according to claim 1, **characterised in that** in the first beam path (3) there are arranged means (6) with which the light beam bundle may be temporarily released or interrupted.

3. A device according to claim 1, **characterised in that** the guiding of the light of the second beam path (7) to the fibre-optic (18) is effected by way of a part-transparent mirror (9).

4. A device according to claims 3, **characterised in that** behind the mirror (9) there is arranged a spectrometer (11) and that in front of the spectrometer (11) there are arranged means (12) with which the light beam bundle in front of the spectrometer (11) may be temporarily released or interrupted.

5. A device according to claim 2 and 4, **characterised in that** the means (6, 12) for the temporary release and interruption of the light beam bundle consists of a first (6) and second chopper wheel (12) which may be driven synchronously, and that both chopper wheels have opaque surfaces which over a region defined in each case comprise recesses (15, 16).

6. A device according to claim 5, **characterised in that** the recesses (15, 16) of both chopper wheels (6, 12) are formed complementarily to one another such that a covering or opaque region of the second chopper wheel corresponds to the removed region (15) of the first chopper wheel (6) and that a removed region (16) of the second chopper wheel (12) corresponds to the covering or opaque region of the first chopper wheel (6).

7. A device according to one of the claims 1 to 6, **characterised in that** the element widening the bundle opening for the light beam bundle entering into the fibre-optic (18) from the first beam path (3) consists of a strongly focusing optical element, for example the lens (27) with a short focal length.

8. A device according to one of the claims 1 to 6, **characterised in that** the element widening the bundle opening, for the light beam bundle entering into the fibre-optic (18) from the first beam path (3) is realised with an oblique coupling-in of the light beam bundle from the beam path (3) into the fibre-optic (18).

9. A device according to one of the claims 1 to 6, **characterised in that** the element widening the bundle opening, for the light beam bundle entering into the fibre-optic from the first beam path (3) consists of an element of claims 7 and 8 alone or of a combination of these elements.

10. A device according to one of the claims 1 to 6, **characterised in that** the element limiting the bundle opening, for the light beam bundle entering into the fibre-optic (18) from the second beam path (7) consists of an illumination means (2a) which emits a parallel light beam bundle with a small diameter.

11. A device according to one of the claims 1 to 6, **characterised in that** the element limiting the bundle opening, for the light beam bundle entering into the fibre-optic (18) from the second beam path (7) consists of a telescope (8) with a suitable focal length ratio of its two lenses.

12. A device according to one of the claims 1 to 6, **characterised in that** the element limiting the bundle opening, for the light beam bundle entering into the fibre-optic (18) from the second beam path (7) consists of an aperture-limiting diaphragm in the beam path (7).

13. A device according to claim 12, **characterised in that** the aperture limiting diaphragm is realised by the limited extension of at least one of the optical elements in the beam path (7).

14. A device according to one of the claims 1 to 6, **characterised in that** the element limiting the bundle opening, for the light beam bundle entering into the fibre-optic (18) from the second beam path (7) consists of one of the elements of the claims 10 to 13 alone or a combination of these elements.

15. A device according to one of the claims 1 to 14, **characterised in that** the fibre-optic (18) leading the light to the endoscope (5) consists of a fluid fibre-optic with a high transmission in the fluorescence excitation band.

16. A device according to one of the claims 1 to 14, **characterised in that** the fibre-optic (18) consists of a fibre or fibre bundle with a high transmission in the fluorescence excitation band.

17. A device according to one of the claims 15 and 16, **characterised in that** the fibre-optic (18) runs up to the distal end of the endoscope (5).

18. A device according to one of the claims 1 to 6, **characterised in that** the second illumination means (2a) is a laser.

19. A device according to one of the claims 1 to 6, **characterised in that** the second illumination means (2a) mixed-gas lamp.

20. A device according to one of the claims 1 to 6, **characterised in that** the second illumination means (2a) is a light diode or an array of light diodes.

21. A device according to on of the claims 1 to 6, **characterised in that** the second illumination means (2a) is a short arc lamp.

22. A device according to one of the claims 1 to 6, **characterised in that** the second illumination means (2a) is a filament lamp.

## Revendications

1. Dispositif d'imagerie et de diagnostic spectroscopique de tissu (1) avec l'utilisation alternée ou combinée de trois modes de diagnostic, à savoir un mode A pour le diagnostic d'imagerie à lumière blanche, un mode B pour le diagnostic d'imagerie à fluorescence et un mode C pour le diagnostic spectroscopique à fluorescence, le dispositif présentant un moyen d'éclairage (2), dont la lumière est injectée sous la forme d'un faisceau de rayons par une trajectoire de rayons (3) dans un conducteur optique (18) aboutissant à un endoscope (5), **caractérisé en ce qu'**il présente en dehors du premier moyen d'éclairage (2) susmentionné un second moyen d'éclairage (2a) dont la lumière est superposée sous la forme d'un faisceau de rayons au moyen d'une seconde autre trajectoire de rayons (7) avant de quitter l'appareil à l'autre première trajectoire de rayons (3) et injectée ensuite dans le conducteur optique (18), **en ce qu'**un élément élargissant l'ouverture de faisceau pour le faisceau de rayons de lumière arrivant dans le conducteur optique (18), de la première trajectoire de rayons optiques est disposé dans la première trajectoire de rayons (3) et **en ce qu'**un élément limitant l'ouverture de faisceau pour le faisceau de rayons lumineux, arrivant dans le conducteur optique, de la seconde trajectoire de rayons est disposé dans la seconde trajectoire de rayons (7).

2. Dispositif selon la revendication 1, **caractérisé en ce que** dans la première trajectoire de rayons (3) sont disposés des moyens (6) avec lesquels le faisceau de rayons lumineux peut être autorisé ou suspendu de façon momentanée.

3. Dispositif selon la revendication 1, **caractérisé en ce que** le guidage de la lumière de la seconde trajectoire de rayons (7) pour le conducteur optique (18) est effectué au moyen d'un miroir (9) semi-argenté.

4. Dispositif selon la revendication 3, **caractérisé en ce qu'**un spectromètre (11) est disposé derrière le miroir (9) et **en ce qu'**avant le spectromètre (11) sont disposés des moyens (12) avec lesquels le faisceau de rayons lumineux avant le spectromètre (11) peut être momentanément autorisé ou suspendu.

5. Dispositif selon les revendications 2 et 4, **caractérisé en ce que** les moyens (6, 12) pour l'autorisation et la suspension momentanée des faisceaux de rayons lumineux comprennent une première (6) et une seconde roue à vibrateur (12) qui peuvent être entraînées de façon synchrone et **en ce que** les deux roues à vibrateur (6, 12) présentent des surfaces opaques qui présentent les évidements (15, 16) au-dessus d'une zone respectivement définie.

6. Dispositif selon la revendication 5, **caractérisé en ce que** les évidements (15, 16) des deux roues à vibrateur (6, 12) sont conçus de façon complémentaire l'un par rapport à l'autre, **en ce qu'**une zone recouvrante et opaque de la seconde roue à vibrateur (12) correspond à la zone (15) évidée de la première roue à vibrateur (6) et **en ce qu'**une zone (16) évidée de la seconde roue à vibrateur (12) correspond à la zone recouvrante et opaque de la première roue à vibrateur (6).

7. Dispositif selon l'une quelconque des revendications 1 à 6, **caractérisé en ce que** l'élément élargissant l'ouverture de faisceau pour le faisceau de rayons lumineux entrant dans le conducteur optique (18) et provenant de la première trajectoire de rayons (3) est constitué d'un élément optique de forte focalisation, par exemple la lentille (27) de courte distance focale.

8. Dispositif selon l'une quelconque des revendications 1 à 6, **caractérisé en ce que** l'élément élargissant l'ouverture de faisceau pour le faisceau de rayons lumineux entrant dans le conducteur optique (18) et provenant de la première trajectoire de rayons (3) est réalisé avec une injection oblique du faisceau de rayons lumineux provenant de la trajectoire de rayons (3) dans le conducteur optique (18).

9. Dispositif selon l'une quelconque des revendications 1 à 6, **caractérisé en ce que** l'élément élargissant l'ouverture de faisceau pour le faisceau de rayons lumineux entrant dans le conducteur optique (18) et provenant de la première trajectoire de rayons (3) est constitué d'un élément des revendications 7 et 8 uniquement ou d'une combinaison de ces éléments.

10. Dispositif selon l'une quelconque des revendications 1 à 6, **caractérisé en ce que** l'élément délimitant l'ouverture de faisceau pour le faisceau de rayons lumineux entrant dans le conducteur optique (18) et provenant de la seconde trajectoire de rayons (7) est constitué d'un moyen d'éclairage (2a), qui émet un faisceau parallèle de rayons lumineux avec un faible diamètre.

11. Dispositif selon l'une quelconque des revendications 1 à 6, **caractérisé en ce que** l'élément délimitant l'ouverture de faisceau pour le faisceau de rayons lumineux entrant dans le conducteur optique (18) et provenant de la seconde trajectoire de rayons (7) est constitué d'un télescope (8) avec un rapport de focales approprié de ses deux lentilles.

12. Dispositif selon l'une quelconque des revendications 1 à 6, **caractérisé en ce que** l'élément délimitant l'ouverture de faisceau pour le faisceau de rayons lumineux entrant dans le conducteur optique (18) se compose de la seconde trajectoire de rayons (7) provenant d'un cache délimitant l'ouverture dans la trajectoire de rayons (7).

13. Dispositif selon la revendication 12, **caractérisé en ce que** le cache délimitant l'ouverture est réalisé par l'extension limitée d'au moins l'un des éléments optiques dans la trajectoire de rayons (7).

14. Dispositif selon l'une quelconque des revendications 1 à 6, **caractérisé en ce que** l'élément délimitant l'ouverture de faisceau pour le faisceau de rayons lumineux entrant dans le conducteur optique (18) et provenant de la seconde trajectoire de rayons (7) est constitué de l'un des éléments des revendications 10 à 13 uniquement ou d'une combinaison de ces éléments.

15. Dispositif selon l'une quelconque des revendications 1 à 14, **caractérisé en ce que** le conducteur optique (18) amenant la lumière à l'endoscope (5) comprend un conducteur optique à fluide avec une transmission élevée dans la bande d'excitation de fluorescence.

16. Dispositif selon l'une quelconque des revendications 1 à 14, **caractérisé en ce que** le conducteur optique (18) est à base d'une fibre ou d'un faisceau de fibres présentant une transmission élevée dans la bande d'excitation de fluorescence.

17. Dispositif selon l'une quelconque des revendications 15 et 16, **caractérisé en ce que** le conducteur optique (18) est disposé jusqu'à l'extrémité distale de l'endoscope (5).

18. Dispositif selon l'une quelconque des revendications 1 à 6, **caractérisé en ce que** le second moyen d'éclairage (2a) est un laser.

19. Dispositif selon l'une quelconque des revendications 1 à 6, **caractérisé en ce que** le second moyen d'éclairage (2a) est une lampe à gaz mixte.

20. Dispositif selon l'une quelconque des revendications 1 à 6, **caractérisé en ce que** le second moyen d'éclairage (2a) est une diode électroluminescente ou un ensemble de diodes électroluminescentes.

21. Dispositif selon l'une quelconque des revendications 1 à 6, **caractérisé en ce que** le second moyen d'éclairage (2a) est une lampe à arc court.

22. Dispositif selon l'une quelconque des revendications 1 à 6, **caractérisé en ce que** le second moyen d'éclairage (2a) est une lampe à incandescence.
